(19) Europäisches Patentamt
European Patent Office
Office européen des brevets

(11) Publication number: 0 260 719 A1

(12) **EUROPEAN PATENT APPLICATION**

(21) Application number: 87113722.0

(22) Date of filing: 18.09.87

(51) Int. Cl.4: A61K 31/557, A61K 47/00, A61K 9/70

(30) Priority: 19.09.86 JP 223229/86

(43) Date of publication of application: 23.03.88 Bulletin 88/12

(84) Designated Contracting States: CH DE FR GB LI SE

(71) Applicant: TEIKOKU SEIYAKU KABUSHIKI KAISHA
567 Sanbonmatsu Ouchi-cho
Okawa-gun Kagawa-ken(JP)

(72) Inventor: Ryoji, Konishi
1989-86, Sanbonmatsu Ochi-cho
Okawa-gun Kagawa-ken(JP)
Inventor: Akiya, Yamada
1278-1, Sanbonmatsu Ochi-cho
Okawa-gun Kagawa-ken(JP)
Inventor: Noriko, Tsuzuki
558-1, Sanbonmatsu Ochi-cho
Okawa-gun Kagawa-ken(JP)

(74) Representative: Kraus, Walter, Dr. et al
Patentanwälte Kraus, Weisert & Partner
Thomas-Wimmer-Ring 15
D-8000 München 22(DE)

(54) **Stable prostaglandin-containing composition.**

(57) A stable prostaglandin-containing composition which comprises a physiologically active protaglandin (e.g. $PGE_1$, $PGE_2$, $PFG_{1\alpha}$ and $PFG_{2\alpha}$) in admixture with a vinyl acetate resin in an amount of 10 to 10,000 parts by weight to 1 part by weight of the prostaglandin.

EP 0 260 719 A1

Xerox Copy Centre

## STABLE PROSTAGLANDIN-CONTAINING COMPOSITION

This invention relates to a stable prostaglandin-containing composition, more particularly to a pharmaceutical composition comprising a physiologically active prostaglandin and vinyl acetate resin, wherein the porstaglandin is contained stably.

### Technical Background and Prior Art

The physiological prostaglandin means prostaglandins (abbreviated "PG") which are contained in the living body and have physiological activities and includes so-called primary prostaglandins, such as prostaglandin $E_1$ (abbreviated "$PG_1$"), prostaglandin $E_2$ (abbreviated "$PGE_2$"), prostanglandin $F_{1\alpha}$ - (abbreviated "$PGF_{1\alpha}$), prostaglandin $F_{2\alpha}$(abbreviated "$PGF_{2\alpha}$"), and their derivatives.

It is known that these compounds are widely distributed within the living body and show hormone like activities and exhibit various pharmacological activities, such as labor inducing activity, vasodilating activity, gastric secretion inhibitory activity, platelet agglutination inhibitory activity, cytostatic activity, etc. in a very small amount, and hence, it has been expected that it would be useful as a medicament.

However, prostaglandins are generally unstable and are easily decomposed by acids, alkaline materials, heat, light, etc. Particularly, PGE ($PGE_1$, $PGE_2$) have carbonyl group within the 5-membered cyclic group thereof, and hence, as is shown in the following scheme, they are easily dehydrated to be converted into PGA ($PGA_1$, $PGA_2$) under an acidic condition, and further are isomerized under an alkaline condition to be converted into PGB ($PGB_1$, $PGB_2$).

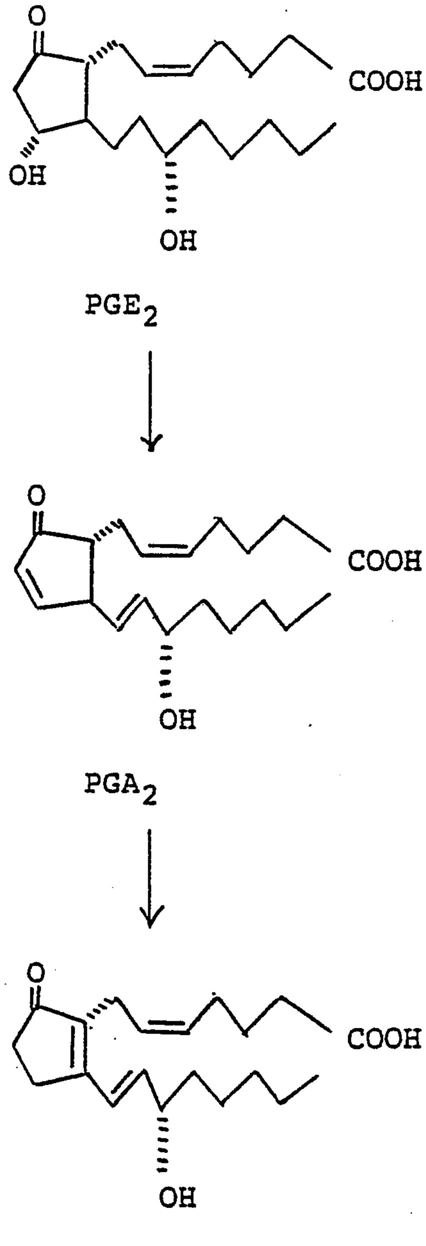

$PGE_2$

$PGA_2$

$PGB_2$

They say that crystalline prostaglandins can usually be kept stably for about one year when they are kept in a frozen state at -20°C, but unless they are stable under severer conditions, it is difficult to develop as a medicament for commercial purpose.

Recently, various studies have been done to find a method for stabilizing prostaglandins. For instance, there are suggested some methods for stabilizing prostaglandins comprising adding a prostaglandin to an aqueous solution of a cellulose derivative (e.g. methyl cellulose, carboxymethyl cellulose, hydroxyethyl cellulose, etc.) and lyophilizing the solution. However, even by these methods, the prostaglandins are not necessarily sufficiently stabilized.

Summary Description of the Invention

Under the circumstance, the present inventors have intensively studied to find an improved method for stabilizing prostaglandins by admixing them with various substances, and have found that when a vinyl acetate resin which is usually used as an additive for foods is admixed with prostaglandins, it is effective for preventing the undesirable decomposition of the prostaglandins and hence for improving the storage stability thereof.

An object of the invention is to provide a stable prostaglandin-containing composition. Another object of the invention is to provide an improved method for stabilizing physiologically active prostaglandins by admixing a vinyl acetate resin. These and other objects and advantages of the invention will be apparent to those skilled in the art from the following description.

Detailed Description of the Invention

The composition of the invention comprises a physiologically active prostaglandin in admixture with a vinyl acetate resin. The vinyl acetate is incorporated in an amount of 10 to 10,000 parts by weight to 1 part by weight of the prostaglandin. The composition can be prepared by dissolving vinyl acetate resin in an organic solvent, such as methanol, acetone, ethyl acetate, etc., or a mixture of two or more thereof, and then subjecting to drying under reduced pressure or under aeration.

The composition thus prepared may be formed into a sheet-like material after being optionally incorporated with an appropriate plasticizer (e.g. polyethylene glycol 400, castor oil, 1,3-butanediol, glycerin ester of fatty acids, triacetine, triethyl citrate, etc.), or into a conventional pharmaceutical preparation, such as tablets, capsules and any other various pharmaceutical preparations. The pharmaceutical preparations can be prepared by a conventional method using conventional pharmaceutically acceptable carriers or diluents, for example, crystalline cellulose, gluten, starch, lactose, and the like.

The vinyl acetate resin used in the present invention includes any commercially available vinyl acetate resin, particularly vinyl acetate resin having a molecular weight 120,000 to 500,000 (a polymerization degree of 200 to 800).

The prostaglandin is contained in the composition in a physiologically effective amount, usually in an amount of from 1 $\mu$g to 5 mg in a dosage unit.

The composition of the present invention has excellent stability of the active prostaglandin which has been confirmed by various tests for stability. Thus, the present invention can provide a composition containing stably the active prostaglandin.

The present invention is illustrated by the following Examples and Experiments, but should not be construed to be limited thereto.

Example 1

Vinyl acetate resin (polymerization degree: 210, Kanebo KBK Resin 0-2, 10 g) is dissolved in ethanol (3.3 g), and thereto is added $PGE_2$ (0.0846 g). The mixture is well homogenized by stirring sufficiently, and then de-aerated. The resulting mixture is spread on a substrate and dried to give a sheet containing $PGE_2$ having a thickness of about 100 $\mu$m.

Example 2

Vinyl acetate resin (polymerization degree: 210, Kanebo KBK 0-2, 1.5 g) is dissolved in methanol (100 ml). To the solution (1 ml) is added a solution of $PGE_2$ (100 $\mu$g) in methanol (10 $\mu$l), and the mixture is well homogenized by stirring, and then, the solvent is distilled off under nitrogen gas to give a $PGE_2$ -vinyl acetate resin composition.

In the same manner as described above, the compositions containing $PGE_1$, $PGF_{2\alpha}$, or $PFG_{1\alpha}$ in admixture with a vinyl acetate resin are prepared.

Experiment 1

As the test sample, there were used the sheet prepared in Example 1 and a sheet containing $PGE_2$ in admixture with other high molecular compounds in the same manner as described in Example 1, and also a commercially available $PGE_2$ product. The test sample was heated at 100°C for 24 hours, and thereafter, the remaining amount of $PGE_2$ in the test sample was measured by liquid chromatography. The liquid chromatography was carried out by using a column packed with octadecylsilylated silica gel and using as a mobile phase a mixture of $KH_2PO_4$ (9 g/liter) - acetonitrile, where the amount of $PGE_2$ was measured by absorbace at 195 nm, followed by calculating based on the standard curve previously prepared. The results are shown in Table 1.

Table 1 (Heat stability test)

| High molecular weight compounds to be admixed with $PGE_2$ | Remaining percent of $PGE_2$ (%) |
|---|---|
| Vinyl acetate resin (Example 1) | 84.3 |
| Polyvinyl alcohol | 0 |
| Cellulose acetate phthalate | 0 |
| None ($PGE_2$ product per se) | 0 |

Experiment 2

The sheet as prepared in Example 1 was cut in a square of 1 $cm^2$ to give a test sample. The test sample was subjected to a dissolving out test by a rotating basket method as defined Japanese Pharmacopoeia. That is, the test sample was entered into an artificial intestinal liquid (50 ml) and stirred at 25 r.p.m. at 37°C, and after 2, 4 and 6 hours, the amount of $PGE_2$ dissolved out into the liquid was measured in the same manner as in Example 1. The results are shown in Table 2.

Table 2 (Dissolving out test)

| Time for dissolving out (hour) | Dissolving rate (cumulative) (%) |
|---|---|
| 2 | 4.5 |
| 4 | 7.6 |
| 6 | 9.3 |

Experiments 3

The compositions containing $PGE_2$, $PGE_1$, $PGF_{2\alpha}$ or $PGF_{1\alpha}$ and the corresponding commercially available prostaglandin products were used as the test samples. The test sample was heated at 100°C for 24 hours, and the remaining amount of the PGs in each sample was measured in the same manner as described in Example 1. The results are shown in Table 3.

Table 3 (Heat stability test)

| Test samples | Remaining percent of PG (%) |
|---|---|
| $PGE_2$ product | 0 |
| Vinyl acetate resin-$PGE_2$ composition | 86.2 |
| $PGE_1$ product | 0 |
| Vinyl acetate resin-$PGE_1$ composition | 86.1 |
| $PGF_{2\alpha}$ product | 29.2 |
| Vinyl acetate resin-$PGF_{2\alpha}$ composition | 100.0 |
| $PGF_{1\alpha}$ product | 43.8 |
| Vinyl acetate resin-$PGF_{1\alpha}$ composition | 100.0 |

**Claims**

1. A stable prostaglandin-containing composition comprising an effective amount of a physiologically active prostaglandin in admixture with a vinyl acetate resin.
2. The composition according to claim 1, wherein the vinyl acetate resin is incorporated in an amount of 10 to 10,000 parts by weight to 1 part of the prostaglandin.
3. The composition according to claim 1, wherein the prostaglandin is a member selected from the group consisting of prostaglandin $E_1$, prostaglandin $E_2$, prostaglandin $F_{1\alpha}$ and prostaglandin $F_{2\alpha}$.

European Patent Office

# EUROPEAN SEARCH REPORT

Application Number

EP 87 11 3722

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 96, no. 14, 5th April 1982, page 386, abstract no. 110129p, Columbus, Ohio, US; & JP-A-81 142 208 (ONO PHARMACEUTICAL CO., LTD) 06-11-1981<br>* Abstract * | 1,2 | A 61 K 31/557<br>A 61 K 47/00<br>A 61 K 9/70 |
| X | EP-A-0 050 480 (ONO PHARMACEUTICAL CO.)<br>* Page 5, lines 4,13-20; claims 1,10 * | 1-3 | |
| Y | CHEMICAL ABSTRACTS, vol. 80, no. 5, 11th March 1974, page 299, abstract no. 52394c, Columbus, Ohio, US; & JP-A-73 12 069 (ENSAI CO., LTD) 18-04-1973<br>* Abstract * | 1 | |
| X,Y | CHEMICAL ABSTRACTS, vol. 96, no. 18, 3rd May 1982, page 418, abstract no. 149109g, Columbus, Ohio, US; J.C. McREA et al.: "Prostaglandin releasing polymers - stability and efficacy", & TRANS. - AM. SOC. ARTIF. INTERN. ORGANS 1981, 27, 511-16<br>* Abstract * | 1-3 | **TECHNICAL FIELDS SEARCHED (Int. Cl.4)**<br>A 61 K |
| X | EP-A-0 106 107 (NIPPON K.K.K.)<br>* Page 1, paragraph 2; page 6, line 10; page 9, paragraph 1; claims * | 1-3 | |
| P,X | EP-A-0 200 508 (NITTO ELECTRIC IND. CO.)<br>* Column 2, lines 36-44; column 12, lines 25-26; comparative example 1 * | 1-3 | |

The present search report has been drawn up for all claims

| Place of search | Date of completion of the search | Examiner |
|---|---|---|
| THE HAGUE | 24-11-1987 | BERTE M.J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)